# FASCICULE DE BREVET EUROPEEN

(11) **EP 2 872 054 B1**
(45) Date de publication et mention de la délivrance du brevet: **30.08.2017**
(21) Numéro de dépôt: 13744737.1
(22) Date de dépôt: 12.07.2013
(51) Int. Cl.: A61B 17/16, A61B 17/32, A61C 1/05, A61C 1/07

(54) **MICROMOTEUR CHIRURGICAL À ULTRASONS**
CHIRURGISCHER ULTRASCHALLMIKROMOTOR
ULTRASONIC SURGICAL MICROMOTOR

(30) Priorité: 16.07.2012 FR 1256861
(43) Date de publication de la demande: 20.05.2015
(73) Titulaire: SOCIETE POUR LA CONCEPTION DES APPLICATIONS DES TECHNIQUES ELECTRONIQUES, 33700 Merignac (FR)
(72) Inventeur: DIERAS, Francis, 33000 Bordeaux (FR); REGERE, Pascal, 33290 Blanquefort (FR)
(74) Mandataire: Healy, Erwan
(86) Numéro de dépôt international: PCT/FR2013/051670
(87) Numéro de publication internationale: WO 2014/013164

(56) Documents cités:
- EP-A1- 0 807 412
- US-A- 5 722 945
- US-A1- 2002 138 080
- US-B1- 6 204 592

## Description

### Arrière-plan de l'invention

La présente invention concerne des appareils de traitement chirurgical et, plus particulièrement, des pièces à main chirurgicales destinées au traitement de tissus biologiques (émail, dentine, muqueuse, os ...) ou de prothèses implantées telles que des implants, des prothèses ou tous autres dispositifs implantables actifs (valves cardiaques...).

L'invention s'applique tout particulièrement à la découpe et/ou au forage de tissus biologiques de densités variables situés à proximité de structures anatomiques fragiles à préserver telles qu'une membrane ou un organe vital par exemple.

De manière conventionnelle, lorsque l'on pratique par exemple une ostéotomie (e.g. découpe d'un os au niveau du genou), on utilise en général une pièce à main chirurgicale munie d'un micromoteur entrainant une fraise, le mouvement rotatif de cette fraise permettant d'entamer le tissu osseux avec une grande efficacité. Le même type de pièce à main est fréquemment utilisé lorsque l'on souhaite forer ou altérer une cavité destinée à recevoir un implant dentaire ou orthopédique.

Cette technique opératoire est relativement efficace mais comporte cependant certains risques du fait notamment de la vitesse de rotation de la fraise qui est parfois difficilement contrôlable, ceci pouvant être la source de lésions irréversibles des tissus voisins tels qu'un nerf ou une artère. Dans le cas d'un forage implantaire, par exemple, la profondeur de celui-ci doit être particulièrement contrôlée pour ne pas léser les tissus sous-jacents. Dans le cas de l'implantologie dentaire, il s'agit par exemple d'éviter de léser le nerf qui traverse la mandibule ou la membrane sinusienne lors du forage de l'os maxillaire.

Pour surmonter ces inconvénients, des premières solutions ont été proposées. La demande de brevet EP 1235527 décrit par exemple un instrument pour la mise en place d'implants dentaires. Cet instrument est muni d'un dispositif destiné à contrôler à la fois la profondeur et l'angle de forage lors de la mise en place d'un implant dentaire.

La demande de brevet EP1733694 décrit également une pièce à main rotative avec un mécanisme de régulation de pression.

Ces solutions technologiques ne sont toutefois pas toujours satisfaisantes en termes d'efficacité de forage ou de découpe.

De manière connue, l'utilisation des ultrasons dans certaines pièces à main chirurgicales présente des avantages pour ces interventions. Cette technique offre en effet la possibilité de remplacer le mouvement rotatif dangereux du micromoteur par des vibrations ultrasonores qui sont transmises du transducteur piézoélectrique à une sonotrode dont la surface et la géométrie permettent une découpe osseuse efficace tout en étant plus sécurisée. L'opération de coupe tissulaire ou de forage est plus précise et mieux contrôlée par l'opérateur. Au contact des tissus de moindre densité, l'action de l'outil devient moins contondante ce qui diminue considérablement la dangerosité du geste sur les structures anatomiques à préserver.

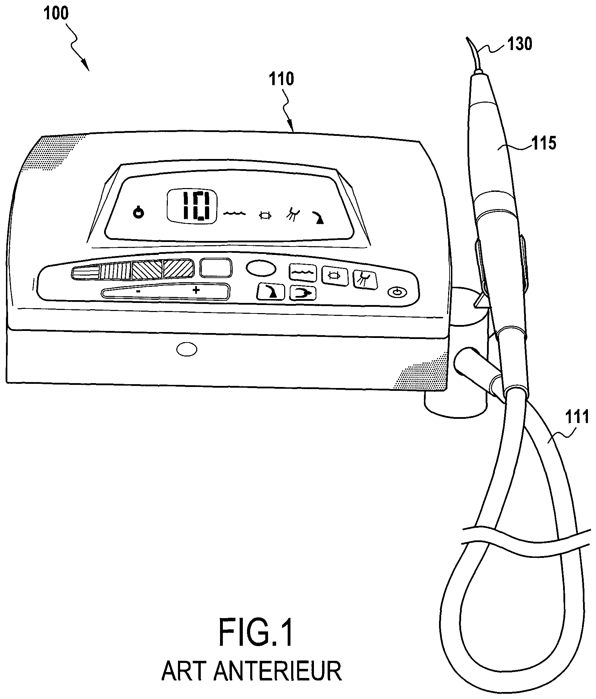

La **figure 1** illustre un exemple de dispositif de contrôle 100 qui comprend un générateur d'ultrasons 110 relié à une pièce à main chirurgicale 115 par un cordon 111. Une sonotrode ou insert ultrasonique 130 est monté sur la partie supérieure ou distale de la pièce à main 115. De façon bien connue, la pièce à main 115 comprend un transducteur (non représenté) formé d'un matériau piézoélectrique et couplé mécaniquement à l'insert 130 de manière à transmettre à ce dernier des vibrations ultrasonores dont les amplitudes sont fonction de la puissance fournie par le générateur d'ultrasons.

Des dispositifs ultrasonores de ce type sont utilisés dans divers domaines chirurgicaux, dont l'ophtalmologie pour la phaco-émulsification du cristallin, la neurochirurgie ou encore l'orthopédie.

Un dispositif de perçage à usage chirurgical est par ailleurs divulgué dans le document de brevet américain référencé US 6,204,592 B1 (cf. figure 1 de ce document). Ce dispositif de perçage comprend à la fois un transducteur piézoélectrique à ultrasons et un micromoteur d'entrainement en rotation.

Le transducteur piézoélectrique est destiné à générer des impulsions vibratoires qui sont transmises à un insert monté à l'extrémité distale de la pièce à main de façon à imprimer à l'insert un mouvement axial de percussion. Le micromoteur permet quant à lui d'entrainer en rotation un ensemble comprenant un arbre rotatif (« horn 16 »). Un instrument est positionné à l'extrémité distale de cet arbre rotatif et le transducteur piézoélectrique est fixé à l'autre extrémité (i.e. proximale) de cet arbre rotatif.

Ce type de pièce à main combine ainsi avantageusement les mouvements vibratoires et rotatifs de façon à optimiser l'action de l'insert sur les tissus biologiques à traiter.

La solution proposée par le document US 6,204,592 B1 présente toutefois des inconvénients majeurs en termes notamment de complexité de construction de la pièce à main. La rotation et les vibrations auxquelles est soumis l'insert sont en outre relativement difficiles à contrôler lorsque l'on opère avec ce type de pièce à main chirurgicale.

Le document EP 0 807 412 A1 daté du 19 novembre 1997 divulgue par ailleurs un dispositif de prélèvement apte à percer des canaux dans du tissu humain au moyen d'un membre de prélèvement à la fois rotatif et déplaçable linéairement.

Il existe donc aujourd'hui un besoin pour une pièce à main chirurgicale offrant un meilleur compromis entre efficacité de découpe et/ou de forage, le contrôle de l'action de l'outil sur les tissus biologiques à traiter et la simplicité de fabrication.

### Objet et résumé de l'invention

A cet effet, la présente invention concerne une pièce à main chirurgicale comprenant :
- un logement destiné à recevoir un insert ;
- un transducteur piézoélectrique à ultrasons comprenant un moteur piézoélectrique disposé entre une contre-masse et une extrémité proximale d'une partie amplificatrice, le transducteur piézoélectrique étant apte à transmettre des ondes ultrasonores à l'insert à partir d'une extrémité distale de ladite partie amplificatrice présente dans le logement ; et
- des moyens d'entraînement en rotation présents dans ledit logement pour entrainer en rotation l'insert ;
dans laquelle les moyens d'entraînement en rotation sont mobiles indépendamment du transducteur piézoélectrique à ultrasons, ledit transducteur piézoélectrique à ultrasons étant statique par rapport à la pièce à main.

Ainsi, le transducteur piézoélectrique à ultrasons reste statique par rapport aux moyens d'entraînement en rotation lorsque ces derniers sont en fonctionnement.

L'intégration de la technologie d'entraînement en rotation et de celle des vibrations ultrasonores permet d'offrir une efficacité optimale en termes de découpe et/ou de forage, par exemple. L'opérateur peut avantageusement réaliser, à l'aide d'une seule et même pièce à main, tous les traitements nécessaires sur les tissus concernés, évitant de passer d'un appareil à l'autre lors de son intervention.

L'invention permet avantageusement aussi bien le réglage optimal de l'amplitude des vibrations ultrasonores que celui de la vitesse de rotation imprimée par les moyens d'entraînement en rotation de manière concomitante ou indépendante.

L'invention permet en outre une transmission optimale des vibrations ultrasonores sans perturbation due à l'éventuelle rotation des moyens d'entraînement en rotation.

En configurant les moyens d'entraînement en rotation pour être mobiles indépendamment du transducteur piézoélectrique à ultrasons et en configurant le transducteur piézoélectrique pour être fixe par rapport à la pièce à main, l'invention permet ainsi d'améliorer le contrôle des vibrations ultrasonores générées par le transducteur piézoélectrique. De plus, la durée de vie du transducteur piézoélectrique s'en trouve sensiblement accrue.

De plus, la construction de la pièce à main selon l'invention est de complexité relativement limitée et présente un robustesse accrue par rapport à d'autres dispositifs comme celui envisagé par exemple dans le document US 6,204,592 B1 commenté ci-avant.

L'énergie nécessaire pour mettre en rotation l'insert est également limitée du fait qu'aucun des éléments du transducteur piézoélectrique à ultrasons ne tournent avec les moyens d'entraînement en rotation.

L'invention s'applique donc tout particulièrement à la découpe et/ou au forage de tissus biologiques de densités variables situés à proximité de structures anatomiques fragiles à préserver telles qu'une membrane, un nerf ou un organe vital par exemple. C'est le cas par exemple lors des laminectomies dans la chirurgie du rachis qui consistent à retirer une ou plusieurs lames vertébrales à proximité de la moelle épinière ou de la base des nerfs crâniens.

Selon un premier mode de réalisation, les moyens d'entraînement en rotation comprennent un micromoteur relié au logement par l'intermédiaire d'un arbre de transmission.

Selon une première variante, l'axe de rotation des moyens d'entraînement en rotation dans le logement est perpendiculaire à une partie distale de l'arbre de transmission. Cette configuration permet de donner une allure coudée à la pièce à main, améliorant ainsi l'ergonomie pour l'opérateur pour certains types d'intervention où l'accès à la zone à traiter est difficile.

Selon une deuxième variante, l'axe de rotation des moyens d'entraînement en rotation dans le logement est parallèle à une partie distale de l'arbre de transmission. Cette configuration droite de la pièce à main peut être particulièrement adaptée pour certains types d'intervention, comme pour le traitement de la colonne vertébrale par exemple.

Cette deuxième variante est également particulièrement adaptée à l'intervention de Chiari en chirurgie pédiatrique lors d'une dysplasie luxante de la hanche nécessitant une ostéotomie du bassin pour loger la tête fémorale. Le champ opératoire se caractérise par une grande exiguïté avec le nerf sciatique et l'artère fessière à la fin du trajet de l'ostéotomie ce qui nécessite de grandes précautions opératoires.

L'innocuité de l'ostéotomie ultrasonore a par ailleurs été démontrée par des expérimentations dont l'observation anatomo-pathologique n'a pas fait apparaitre de lésion cellulaires à proximité de la coupe autant sur le périoste, l'endoste, les cellules ostéocytaires ou les cellules présentes dans la vascularisation intra-osseuse.

Toujours dans cette deuxième variante, le transducteur piézoélectrique à ultrasons peut être configuré pour transmettre les ondes ultrasonores au logement par l'intermédiaire des moyens d'entraînement en rotation.

Selon un deuxième mode de réalisation, les moyens d'entraînement en rotation comprennent une turbine à air et un conduit de transmission, la turbine étant apte à entrer en rotation sous l'action d'un flux de gaz (de l'air par exemple) projeté via ledit conduit de transmission.

Selon une première variante de ce deuxième mode, l'axe de rotation des moyens d'entraînement en rotation dans le logement est perpendiculaire à une partie distale du conduit de transmission. Comme indiqué précédemment, cette configuration permet de donner une allure coudée à la pièce à main, améliorant ainsi l'ergonomie pour l'opérateur pour certains types d'intervention où l'accès à la zone à traiter est difficile.

Selon une deuxième variante de ce deuxième mode, l'axe de rotation des moyens d'entraînement en rotation dans le logement est parallèle à une partie distale du conduit de transmission. Cette configuration droite de la pièce à main peut également être particulièrement adaptée pour certains types d'intervention, comme pour le traitement de la colonne vertébrale par exemple.

Dans un mode de réalisation particulier de l'invention, le transducteur piézoélectrique à ultrasons comprend un élément de transmission vibratoire en céramique aménagé à l'extrémité distale de la partie amplificatrice de façon à transmettre les ondes ultrasonores dans le logement.

Dans un mode de réalisation particulier, la partie amplificatrice présente un étranglement dont la position le long de la partie amplificatrice est fixée de façon à maximiser l'amplitude des mouvements vibratoires de l'extrémité distale de la partie amplificatrice.

Dans un mode de réalisation particulier, la pièce à main de l'invention comprend en outre un insert disposé en partie dans le logement de façon à pouvoir coopérer avec le transducteur piézoélectrique à ultrasons et avec les moyens d'entraînement en rotation.

De manière préférée, en positon de travail, l'insert est disposé de façon à ce que son extrémité proximale soit en contact avec l'extrémité distale de la partie amplificatrice et que le corps de l'insert puisse être entraîné en rotation par les moyens d'entraînement en rotation.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
- la figure 1 commentée ci-avant représente schématiquement un exemple de dispositif de contrôle connu de l'art antérieur ;
- la figure 2 représente schématiquement en vue de coupe longitudinale une pièce à main chirurgicale selon un premier mode de réalisation de l'invention ;
- la figure 3 représente schématiquement une vue de dessus de la pièce à main de la figure 2 ;
- les figures 4A et 4B représentent des variantes de forme de la partie amplificatrice du transducteur piézoélectrique à ultrasons représenté en figure 2 ;
- la figure 5 représente schématiquement en vue de coupe longitudinale une pièce à main chirurgicale selon un deuxième mode de réalisation de l'invention ;
- les figures 6 et 7 représentent en vue de coupe des variantes des pièces à main des figures 2 et 5 respectivement ;
- la figure 8 représente en vue de coupe une autre variante de la pièce à main chirurgicale de la figure 2 ; et
- les figures 9A à 9I représentent schématiquement des exemples d'inserts avec lesquels la pièce à main selon l'invention est susceptible d'être équipée.

### Description détaillée de modes de réalisation de l'invention

La présente invention concerne une pièce à main chirurgicale pour le traitement (découpe, forage...) de tissus biologiques de différentes densités au moyen d'un instrument (ou insert).

Afin de pallier les inconvénients des dispositifs de l'art antérieur, la pièce à main selon l'invention intègre avantageusement deux technologies distinctes : la vibration ultrasonore de l'insert afin de lui imprimer un mouvement de percussion, d'une part, et l'entraînement en rotation de l'insert à l'aide de moyens d'entraînement en rotation appropriés, d'autre part.

Cette pièce à main est ainsi apte à imprimer à l'outil de découpe et/ou de forage (i.e. à l'insert) un mouvement rotatif et un mouvement en percussion et ce, de façon alternée ou simultanée.

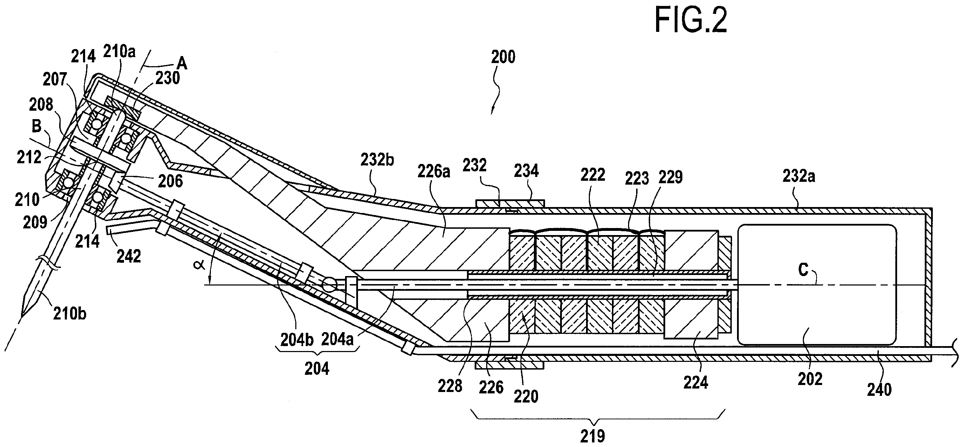

Une pièce à main chirurgicale 200 selon un premier mode de réalisation de l'invention est à présent décrite en référence aux **figures 2****,** **3, 4A et 4B****.**

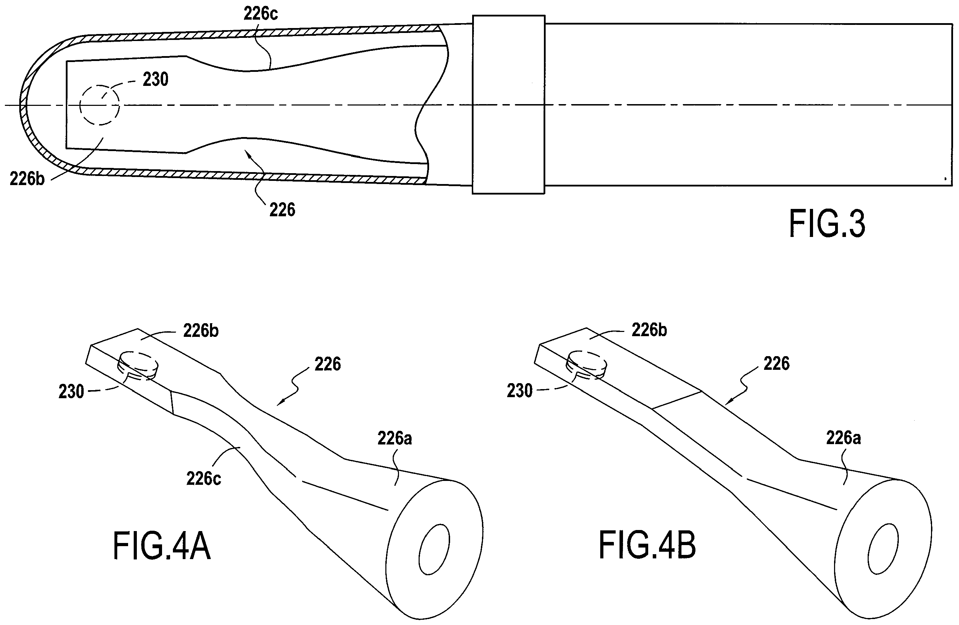

Plus spécifiquement, la **figure 2** représente de façon schématique une vue en coupe latérale de la pièce à main chirurgicale 200. La **figure 3** représente une vue de dessus de cette même pièce à main 200.

La pièce à main chirurgicale 200 comprend un micromoteur 202 situé dans la partie proximale de la pièce à main. Ce micromoteur 202 est couplé mécaniquement à la partie proximale 204a d'un arbre de transmission 204 s'étendant dans le corps de la pièce à main 202.

Dans cet exemple, l'arbre de transmission 204 comprend en outre une partie distale 204b couplée mécaniquement à la partie proximale 204a par un cardan.

La partie distale 204b de l'arbre de transmission 204 présente dans ce cas particulier un angle α non nul avec la partie proximale 204a (angle entre les axes de rotation B et C en figure 2). Comme indiqué plus en détail ultérieurement, cet angle α peut être nul dans d'autres modes de réalisation de l'invention.

La tête de la partie distale 204 de l'arbre de transmission 204 correspond ici à un engrenage 206 coopérant avec une couronne crantée 208, l'engrenage 206 et la couronne crantée 208 étant dans ce cas perpendiculaires l'un par rapport à l'autre.

La couronne crantée 208 est disposée dans un logement 207, ce dernier étant arrangé de façon à pouvoir accueillir un insert 210 destiné à traiter (découpe, forage etc.) des tissus biologiques. La couronne crantée 208 est munie dans cet exemple d'un dispositif de fixation 212 permettant de fixer dans le logement 207 un insert 210 approprié selon le traitement à effectuer.

Le logement 207 est ici formé par une cavité située à l'extrémité distale de la pièce à main 200 et qui débouche sur l'extérieur de la pièce à main via l'orifice 209. Ce logement 207 comprend notamment des roulements à bille 214 permettant à l'insert 210 de tourner librement selon l'axe de rotation A dans le logement 207 lorsque l'insert est entraîné en rotation via l'arbre de transmission 204 par le micromoteur 202.

L'ensemble comprenant ici le micromoteur 202, l'arbre de transmission 204, et la couronne crantée 208 forme un exemple de moyens d'entraînement en rotation selon l'invention. On comprendra cependant qu'il ne s'agit que d'un exemple de mise en oeuvre, d'autres variantes étant envisageables dans le cadre de l'invention, dont certaines seront décrites ultérieurement.

La pièce à main chirurgicale 200 comprend en outre un transducteur piézoélectrique à ultrasons 219 situé dans cet exemple entre le micromoteur 202 et l'extrémité distale de la pièce à main 200.

Le transducteur piézoélectrique à ultrasons 219 comprend un moteur piézoélectrique 220, une contre-masse 224 et une partie amplificatrice 226. Plus précisément, le moteur piézoélectrique 220 comporte une pluralité de disques piézoélectriques 222 en céramique entre lesquels sont intercalés des contacts électriques 223 de façon à pouvoir alimenter électriquement le moteur piézoélectrique 220. L'empilement de disques piézoélectriques 222 est contraint mécaniquement de part et d'autre par la partie amplificatrice 226 (côté distal) et par la contre-masse 224 (côté proximal).

La partie amplificatrice 226 et la contre-masse 224 sont ici reliées par une tige de précontrainte 228 axiale (celle-ci étant maintenue par un écrou de mise en contrainte par exemple) qui est elle-même traversée de bout en bout par un conduit central 229. Le conduit 229 traverse ainsi la contre-masse 224, les disques piézoélectriques 222 (avec leur contact électriques 223), et partiellement la partie amplificatrice 226 de façon à y laisser passer librement l'arbre de transmission 204.

Le moteur piézoélectrique 220 est apte à générer des vibrations ultrasonores sous l'effet d'une alimentation électrique appliquée au moyen des contacts électriques 223. Le fonctionnement et la configuration générale d'un transducteur piézoélectrique à ultrasons étant connus en soi, ceux-ci ne seront pas davantage décrits dans la suite de ce document.

Le transducteur piézoélectrique à ultrasons 219 constitue donc ici des moyens de génération de vibrations ultrasonores.

Les vibrations ultrasonores générées par le transducteur piézoélectrique 219 (lorsque celui-ci est actionné) sont transmises jusqu'au logement 207 par l'intermédiaire de la partie amplificatrice 226, et en particulier à partir de l'extrémité distale 226b de la partie amplificatrice 226 qui est présente dans le fond du logement 207.

Comme représenté en **figure 4A**, la partie amplificatrice 226 présente dans cet exemple un angle α non nul entre ses parties distale et proximale afin de se conformer à la forme coudée de la pièce à main 200. Cette forme coudée de la pièce à main n'est pas toujours requise mais est particulièrement adaptée pour les traitements de type dentaire, par exemple, car elle répond mieux aux exigences d'ergonomie pour l'accès à certaines zones de traitement.

Comme représenté en **figures 3 et 4A**, la partie amplificatrice 226 présente dans cet exemple un étranglement 226c qui permet le contrôle et l'optimisation de l'amplitude des vibrations ultrasonores auxquelles est soumise l'extrémité distale 226b lorsque le moteur piézoélectrique 220 fonctionne. En ajustant la position de cet étranglement 226c le long de la partie amplificatrice 226 (lors de la conception et de la fabrication de cette partie amplificatrice), il est par exemple possible de maximiser les amplitudes vibratoires de l'extrémité distale 226b de la partie amplificatrice 226. La manière dont le positionnement de l'étranglement est réalisé est connue en soi et ne sera donc pas décrit dans ce document.

On comprendra toutefois que la présence d'un tel étranglement n'est pas obligatoire, comme illustré en **figure 4B** par exemple qui représente une partie amplificatrice 226 rectiligne.

On considère à présent le cas où un insert 210 est inséré dans le logement 207 via l'orifice 209 et placé en position de travail de sorte que l'extrémité proximale 210a (i.e. la base) de cet insert soit en butée contre l'extrémité distale 226b de la partie amplificatrice 226 et que l'extrémité distale 210b de l'insert 210 (i.e. son extrémité travaillante) s'étende à l'extérieur du logement 207 et donc de la pièce à main 200.

Dans le cas présent, la base 210a de l'insert est plus précisément en contact avec un élément de transmission vibratoire 230 aménagé en vis-à-vis dans l'extrémité distale 226b de la partie amplificatrice 226. Cet élément de transmission vibratoire 230 est ici une pièce de céramique sertie dans l'extrémité distale 226b, cette pièce présentant une forme circulaire. D'autres formes (carrée...) sont toutefois envisageables. L'ajout de cette pièce permet d'optimiser la transmission des vibrations ultrasonores à l'insert 210.

On notera qu'une force élastique de rappel est appliquée à l'insert 210 afin de maintenir son extrémité 210a en contact avec l'élément de transmission vibratoire 230. Le maintien de ce contact permet d'assurer une meilleure transmission des vibrations ultrasonores générées par le transducteur piézoélectrique 219 jusqu'à l'insert. L'homme du métier comprendra que le mécanisme (non représenté sur les figures) assurant l'application de cette force de rappel peut être réalisé de différentes manières (à l'aide d'un ressort par exemple), et ne sera donc pas décrit plus en détail dans ce document.

On comprendra cependant que le transducteur piézoélectrique à ultrasons 219 décrit ci-avant ne constitue qu'un exemple de mise en oeuvre, d'autres variantes étant envisageables dans le cadre de l'invention, dont certaines seront décrites ultérieurement.

La pièce à main chirurgicale 200 est ainsi apte à :
- imprimer des mouvements en percussion à l'insert 210 en transmettant à celui-ci des vibrations ultrasonores à partir du transducteur piézoélectrique à ultrasons de l'invention ; et
- entraîner en rotation l'insert 210 suivant l'axe de rotation A du logement 207 à partir des moyens d'entraînement en rotation de l'invention.

Ces deux actions rotation/vibration sont réalisées simultanément ou alternativement sur commande de l'utilisateur à l'aide, par exemple, d'une commande présente sur un dispositif du même type que le dispositif de contrôle 100 représenté en figure 1.

Conformément à l'invention, les moyens d'entraînement en rotation sont mobiles indépendamment du transducteur piézoélectrique à ultrasons, ce dernier étant statique par rapport à la pièce à main.

Ainsi, lorsque l'arbre de transmission 204 est entraîné en rotation par le micromoteur 202, le transducteur piézoélectrique à ultrasons 219 reste statique par rapport à la pièce à main 200. Cette configuration présente des avantages majeurs vis-à-vis par exemple des pièces à main connues dans l'art antérieur.

L'intégration de la technologie d'entraînement en rotation et de celle des vibrations ultrasonores permet d'offrir une efficacité optimale en termes de découpe et/ou de forage, par exemple. L'opérateur peut avantageusement réaliser, à l'aide d'une seule et même pièce à main, tous les traitements nécessaires sur les tissus concernés, évitant de passer d'un appareil à l'autre lors de son intervention.

Concernant par exemple le dispositif du document 6,204,592 B1, le transducteur piézoélectrique et les connexions électriques correspondantes sont solidaires de l'arbre de transmission de sorte que l'arbre de transmission et le transducteur piézoélectrique tournent ensemble. Ce montage impose par conséquent que les disques piézoélectriques du moteur piézoélectrique soient alimentés électriquement via un système d'anneaux conducteurs contraignants (cf. références 54 et 56 en figure 2 de ce document). Ce type de connexions électriques annulaires par frottement est relativement fragile et complexe à monter et son réglage peut s'avérer délicat.

De plus, le montage proposé dans le document 6,204,592 B1 expose le transducteur piézoélectrique à des chocs et des contraintes mécaniques importantes lorsque l'arbre de transmission est en mouvement (en rotation dans le cas présent), ce qui risque de diminuer la durée de vie du transducteur piézoélectrique et de dégrader la qualité des vibrations ultrasonores qu'il génère.

En configurant les moyens d'entraînement en rotation pour être mobiles indépendamment du transducteur piézoélectrique à ultrasons et en configurant le transducteur piézoélectrique pour être fixe par rapport à la pièce à main, l'invention permet ainsi d'améliorer le contrôle des vibrations ultrasonores générées par le transducteur piézoélectrique. De plus, la durée de vie du transducteur piézoélectrique s'en trouve sensiblement accrue.

L'invention permet également d'améliorer le contrôle de la rotation des moyens d'entraînement en rotation, et en particulier, de l'arbre de transmission 204 dans le cas présent. La consommation d'énergie (électrique dans ce cas) est également réduite du fait que le micromoteur n'entraîne pas en rotation le transducteur piézoélectrique 220.

Le réglage de la rotation, d'une part, et des vibrations ultrasonores, d'autre part, est ainsi possible de manière indépendante.

L'invention s'applique donc tout particulièrement à la découpe et/ou au forage de tissus biologiques de densités variables situés à proximité de structures anatomiques fragiles à préserver telles qu'une membrane ou un organe vital par exemple.

Les propriétés physiques de l'insert 210 sont de préférence choisies de façon à ce que l'insert puisse avoir une action contondante (ou tranchante) aussi bien en rotation qu'en vibration. L'insert 210 présente, par exemple, une forme avec des stries verticales et horizontales ou, alternativement, des stries obliques. Ces stries peuvent être diamantée ou non. En variante, il est possible d'utiliser des inserts n'ayant une action qu'en rotation ou en vibration.

L'utilisateur est de préférence capable d'utiliser différents inserts selon le travail à effectuer.

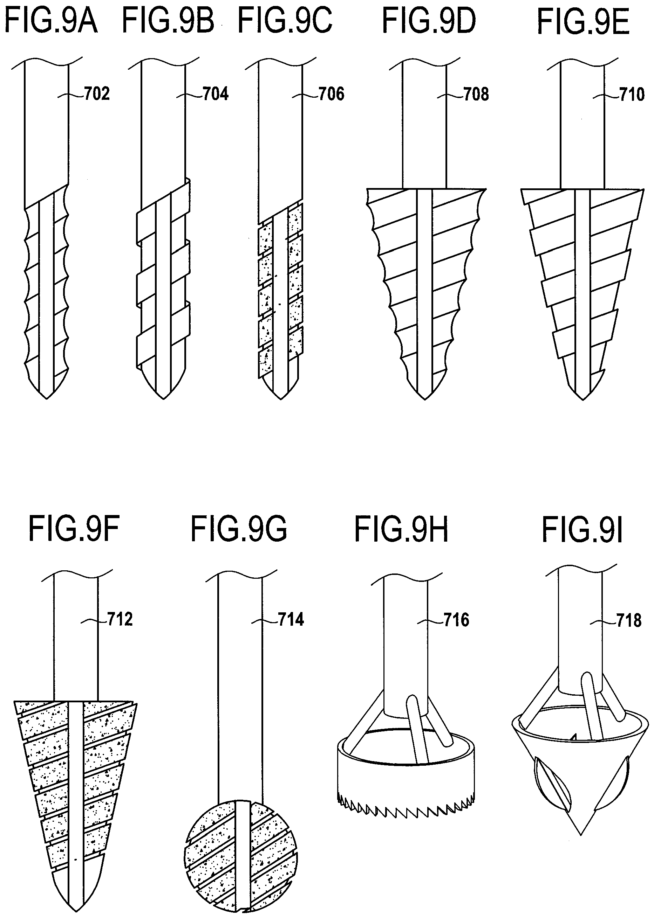

Les **figures 9A à 9I** sont des vues représentant schématiquement des exemples d'inserts qui sont susceptibles d'être installés dans le logement de la pièce à main selon l'invention.

Les figures 9A, 9B et 9C représentent des inserts (notés respectivement 702, 704 et 706) en forme de forets allongés.

Les forets 702 et 704 comportent respectivement des arrêtes contondantes en spirale et une lame contondante en spirale. Le foret 706 est diamanté et comporte des gorges permettant l'évacuation de résidus tissulaires. Ces forets 702, 704 et 706 comportent tout trois un conduit central pour l'évacuation des résidus tissulaires.

On notera que les inserts 702, 74 et 706 seront préférentiellement utilisés pour des interventions de forage ou d'ostéotomie profonde, dans le cas d'un forage implantaire par exemple en chirurgie dentaire.

Les inserts 708, 710 et 712 représentés en figures 9D, 9E et 9F constituent des variantes sous forme conique des inserts 702, 704 et 706, respectivement.

L'insert 714 représenté en figure 9G est diamanté et en forme de boule.

Les inserts 708, 710 et 712 de type conique et l'insert 714 en forme de boule seront utilisés par exemple pour des ostéotomies circulaires de diamètre variable, pour des ostéotomies en forme de géode pour les résections apicales (par exemple en chirurgie dentaire) ou utilisés comme râpe dans le cas par exemple d'ostéophyte ou bec de perroquet vertébraux secondaires à un processus arthrosique.

L'insert 716 illustré en figure 9H est de type trépan ou emporte-pièce et se présente sous la forme d'une couronne circulaire dentée. Ce type d'insert pourra être choisi pour des ostéotomies nécessitant un alésage précis pour l'ajustement d'une fixation prothétique, par exemple, ou encore pour la trépanation de la boîte crânienne en neurochirurgie.

L'insert 718 de la figure 9I est une autre variante d'insert conique comportant cette fois des sortes d'ouïes contondantes s'étendant vers l'extérieur de la surface du cône. Ce type d'insert pourra par exemple être fixé à l'extrémité d'un cathéter en vue d'une artériectomie directionnelle (opération consistant à éliminer l'accumulation de plaque athéromateuse à l'intérieur d'une artère).

On notera par ailleurs que les composants internes de la pièce à main chirurgicale 200, et en particulier ceux correspondant au transducteur piézoélectrique à ultrasons et aux moyens d'entraînement en rotation, sont ici enfermés dans un habillage (ou châssis) 232 comprenant une partie proximale 232a et une partie distale 232b.

Dans cet exemple, la fixation des parties proximales et distales 232a et 232b de l'habillage est renforcé au moyen d'une bague d'accostage 234 à la jonction entre les parties 232a et 232b de l'habillage.

On notera également que la pièce à main 200 comprend dans cet exemple un conduit d'irrigation 240 débouchant sur un orifice 242 placé à proximité de l'orifice 209 du logement 207. Ce conduit d'irrigation 240 permet de faire circuler un liquide longitudinalement dans l'une des deux directions à travers la pièce à main 200, afin d'irriguer si besoin la zone de tissus traitée ou, alternativement, d'évacuer des substances (résidus tissulaires...) provenant de la zone traitée en question.

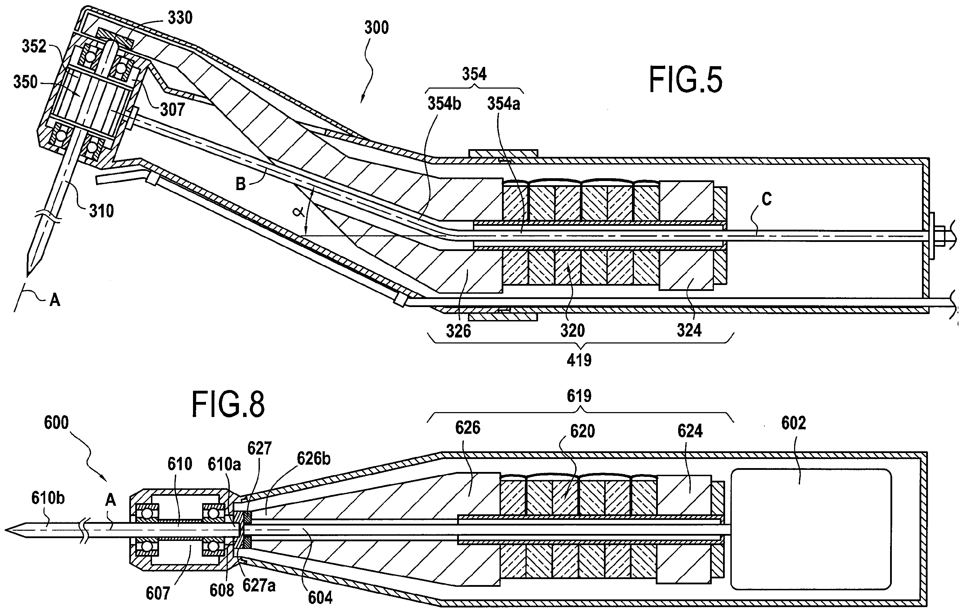

Une pièce à main chirurgicale 300 conforme à un deuxième mode de réalisation de l'invention est à présent décrite en référence à la **figure 5****.**

Les composants de la pièce à main 300 sont en majorité identiques aux composants correspondants de la pièce à main 200 décrite ci-dessus. En l'absence d'indication spécifique, les composants de la pièce à main 300 présentent les mêmes propriétés et répondent au même fonctionnement que les composants correspondants de la pièce à main 200.

Le transducteur piézoélectrique à ultrasons 319 de la pièce à main 300 comprend en particulier un moteur piézoélectrique 320 disposé sous contraintes mécaniques entre une contre-masse 324 et une partie amplificatrice 326, la partie distale de cette dernière étant présente dans un logement 307. Les éléments 319, 320, 324, 326 et 307 sont identiques aux éléments 219, 220, 224, 226 et 207, respectivement, de la pièce à main 200.

La pièce à main 300 diffère de la pièce à main 200 en ce que les moyens d'entraînement en rotation fonctionnent à l'aide d'une turbine à air et non au moyen d'un micromoteur.

Plus spécifiquement, la pièce à main 300 comprend un conduit 354 traversant successivement de part en part la contre-masse 324, le moteur piézoélectrique 320 et la partie amplificatrice 326. Ce conduit 354 débouche sur une turbine à air 350 montée sur pivot dans le logement 307 de façon à pouvoir entraîner en rotation un insert 310 lorsque celui-ci est placé en position de travail dans le logement 307. Cette rotation est obtenue sous l'action d'un fluide sous pression (par exemple un flux de gaz (tel que de l'air par exemple)) projeté via le conduit de transmission 354 contre les pales 352 de la turbine à gaz 350.

On supposera dans cet exemple que la turbine à air 350 est alimentée en air.

Le conduit 354 peut comprendre deux passages distincts, l'un pour l'alimentation du gaz dans la turbine, l'autre pour la récupération du gaz. La conception et le fonctionnement d'une turbine à air sont bien connus de l'homme du métier et ne seront donc pas décrits plus en détail dans ce document.

Les moyens d'entraînement en rotation comprennent donc ici le conduit 354 et la turbine à air 350.

Conformément à l'invention, les moyens d'entraînement en rotation sont ici aussi mobiles indépendamment du transducteur piézoélectrique à ultrasons, le transducteur piézoélectrique à ultrasons étant statique par rapport à la pièce à main 300.

On notera que la pièce à main 300 présente un angle α non nul entre la partie distale 354b et la partie proximale 354a du conduit 354 (i.e. entre les axes B et C) de façon analogue à l'angle formé par les parties proximale 204a et distale 204b du conduit 204 dans la pièce à main 200. La présence de cet angle non nul α implique que la tête travaillante de la pièce à main située à son extrémité distale soit en léger retrait par rapport à la partie proximale de la pièce à main destinée à être prise en main par l'opérateur. Ce retrait de la tête travaillante améliore l'ergonomie de la pièce à main pour certains types d'intervention, notamment dans le domaine dentaire.

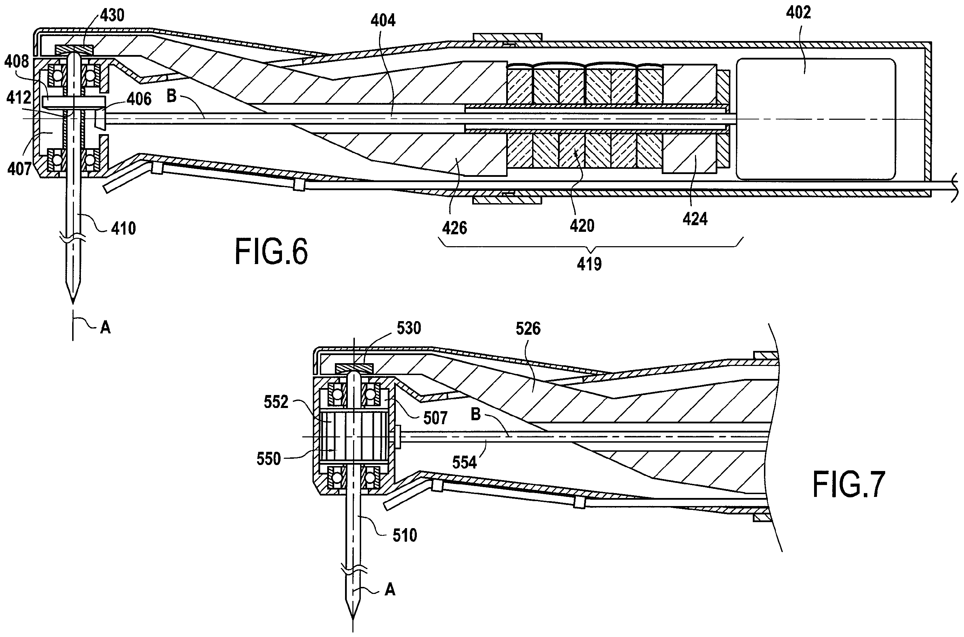

En variante, la conception des pièces à main 200 et 300 peut être modifiée de sorte que cet angle α soit nul, comme cela est représenté respectivement en **figures 6 et 7** (pièces à main 400 et 500, respectivement).

On notera également que les pièces à main décrites précédemment présentent une forme coudée. En effet, dans la pièce à main 200 (respectivement 300) l'axe de rotation A des moyens d'entraînement en rotation dans le logement 207 (respectivement 307) est perpendiculaire à la partie distale 204b (respectivement 354b) de l'arbre de transmission 204 (respectivement du conduit 354). Cette configuration coudée est également présente dans les variantes 400 et 500 représentées en figures 6 et 7.

Comme expliqué précédemment, cette forme coudée offre des avantages en termes d'ergonomie concernant l'accès aux tissus à traiter, notamment dans le cadre des interventions dans le domaine dentaire. Cette forme coudée n'est toutefois pas obligatoire dans le cadre de l'invention.

La **figure 8**, par exemple, représente une pièce à main 600 selon une variante de la pièce à main 200 de la figure 2.

Les composants de la pièce à main 600 sont en majorité identiques aux composants correspondants de la pièce à main 200 décrite ci-dessus. En l'absence d'indication spécifique, les composants de la pièce à main 600 présentent les mêmes propriétés et répondent au même fonctionnement que les composants correspondants de la pièce à main 200.

Le transducteur piézoélectrique à ultrasons 619 de la pièce à main 600 comprend en particulier un moteur piézoélectrique 620 disposé sous contraintes mécaniques entre une contre-masse 624 et une partie amplificatrice 626, la partie distale de cette dernière étant présente dans un logement 607.

Les moyens d'entraînement en rotation de la pièce à main 600 comprennent un micromoteur apte à entraîner en rotation un insert 610 lorsque celui-ci est installé en position de travail dans le logement 607. Cet entraînement en rotation est réalisé par l'intermédiaire d'un arbre de transmission 604 rotatif.

La mise en oeuvre de la figure 8 diffère principalement en ce qu'il s'agit d'une pièce à main droite et non d'allure coudée comme les pièces à main décrites précédemment.

La partie amplificatrice 626 présente ici une symétrie de révolution autour de l'axe principal de la pièce à main 600 (i.e. l'axe de rotation A de l'insert dans le logement 607). Dans cette mise en oeuvre, l'axe de l'arbre de transmission 604 est confondu avec l'axe de rotation A des moyens d'entraînement en rotation dans le logement 607.

Par ailleurs, l'arbre de transmission 604 est dans cet exemple muni à son extrémité distale d'une tête circulaire 608 présente dans le logement 607. Cette tête 608 est configurée de façon à ce que l'on puisse y fixer l'extrémité proximale 610a de l'insert 610 (en la vissant dans un logement prévu à cet effet par exemple).

La tête 608 située à l'extrémité distale de l'arbre de transmission 604 s'appuie contre la surface 627a de l'extrémité distale de la partie amplificatrice 626. Grâce à ce contact physique en bordure du logement 607 entre la tête 608 de l'arbre de transmission 604 et la surface 627a, la partie amplificatrice 626 est apte à transmettre à l'insert 610 les vibrations ultrasonores générées par le moteur piézoélectriques 620.

Des frottements importants peuvent se produire à l'interface entre la tête 608 (mobile en rotation) et l'extrémité distale de la partie amplificatrice 626 (statique). C'est pourquoi, dans cette mise en oeuvre, l'extrémité distale de la partie amplificatrice est constituée d'une pièce annulaire 627 dont les propriétés permettent une bonne transmission par frottement des vibrations ultrasonores à l'insert 610 via la tête 608.

On notera que dans les modes de réalisations précédents, le transducteur piézoélectrique à ultrasons et les moyens d'entraînement en rotation de l'invention sont complètement indépendants les uns des autres. Chacun d'eux coopère avec l'insert de façon indépendante. Dans le cas de la pièce à main 600, en revanche, le transducteur piézoélectrique à ultrasons 619 transmet les ondes ultrasonores dans le logement 607 par l'intermédiaire de la tête 608 (qui fait partie des moyens d'entraînement en rotation).

Des variantes de la pièce à main 600 sont toutefois possibles dans lesquels l'extrémité distale de la partie amplificatrice est directement en contact avec l'insert de sorte que la transmission des vibrations ultrasonores se fasse directement. On peut par exemple modifier la forme de la tête 608 de sorte qu'une partie de l'extrémité distale de la partie amplificatrice 626 touche physiquement l'insert en fonctionnement.

Des variantes en configuration droite de la pièce à main 300 (à turbine à air) par exemple peuvent également être envisagées dans le cadre de l'invention.

Toutes les variantes envisagées ci-avant présentent les mêmes avantages que ceux exposés en relation avec la pièce à main 200.

La pièce à main chirurgicale selon l'invention trouve une application toute particulière dans le traitement de tissus biologiques (émail, dentine, muqueuse, os ...) ou de prothèses implantées telles que des implants, des prothèses ou tous autres dispositifs implantables actifs (valves cardiaques...).

## Revendications

1. Pièce à main chirurgicale (200) comprenant :
- un logement (207) destiné à recevoir un insert (210) ;
- un transducteur piézoélectrique à ultrasons (219) comprenant un moteur piézoélectrique (220) disposé entre une contre-masse (224) et une extrémité proximale (226a) d'une partie amplificatrice (226), le transducteur piézoélectrique étant apte à transmettre des ondes ultrasonores audit insert à partir d'une extrémité distale (226b) de ladite partie amplificatrice (226) présente dans le logement (207) ; et
- des moyens d'entraînement en rotation (202, 204, 208) présents dans ledit logement pour entraîner en rotation ledit insert ;
les moyens d'entraînement en rotation étant mobiles indépendamment du transducteur piézoélectrique à ultrasons (219),
la pièce à main chirurgicale étant **caractérisée en ce que** ledit transducteur piézoélectrique à ultrasons est statique par rapport à la pièce à main (200).

2. Pièce à main selon la revendication 1, dans laquelle les moyens d'entraînement en rotation comprennent un micromoteur (202) relié au logement par l'intermédiaire d'un arbre de transmission (204).

3. Pièce à main selon la revendication 2, l'axe de rotation (A) des moyens d'entraînement en rotation dans ledit logement (207) étant perpendiculaire à une partie distale (204b) de l'arbre de transmission.

4. Pièce à main selon la revendication 2, dans laquelle l'axe de rotation (A) des moyens d'entraînement en rotation dans ledit logement (607) est parallèle à une partie distale de l'arbre de transmission (604).

5. Pièce à main selon la revendication 4, dans laquelle le transducteur piézoélectrique à ultrasons (619) est configuré pour transmettre les ondes ultrasonores audit logement (607) par l'intermédiaire des moyens d'entraînement en rotation.

6. Pièce à main selon la revendication 1, dans laquelle les moyens d'entraînement en rotation comprennent une turbine à air (350) et un conduit de transmission (354), ladite turbine étant apte à entrer en rotation sous l'action d'un flux de gaz projeté via ledit conduit de transmission.

7. Pièce à main selon la revendication 6, l'axe de rotation (A) des moyens d'entraînement en rotation dans ledit logement (307) étant perpendiculaire à une partie distale (354b) du conduit de transmission (354).

8. Pièce à main selon la revendication 6, l'axe de rotation (A) des moyens d'entraînement en rotation dans ledit logement étant parallèle à une partie distale du conduit de transmission.

9. Pièce à main selon l'une quelconque des revendications 1 à 8, dans laquelle le transducteur piézoélectrique à ultrasons (219) comprend un élément de transmission vibratoire (230) en céramique aménagé à l'extrémité distale (226b) de la partie amplificatrice (226) de façon à transmettre les ondes ultrasonores dans ledit logement (207).

10. Pièce à main selon l'une quelconque des revendications 1 à 9, dans laquelle la partie amplificatrice (226) présente un étranglement (226c) dont la position le long de la partie amplificatrice est fixée de façon à maximiser l'amplitude des mouvements vibratoires de l'extrémité distale (226b) de la partie amplificatrice.

11. Pièce à main selon l'une quelconque des revendications 1 à 10 comprenant en outre un insert (210) disposé en partie dans le logement (207) de façon à pouvoir coopérer avec le transducteur piézoélectrique à ultrasons (219) et avec les moyens d'entraînement en rotation (202, 204, 208).

12. Pièce à main selon la revendication 11, dans laquelle l'insert (210) est disposé de façon à ce que son extrémité proximale (210a) soit en contact avec l'extrémité distale (226b) de la partie amplificatrice (226) et que le corps de l'insert (210) puisse être entraîné en rotation par les moyens d'entraînement en rotation (202, 204, 208).

## Patentansprüche

1. Chirurgisches Handstück (200), umfassend:
- eine Aufnahme (207), die dazu bestimmt ist, einen Einsatz (210) aufzunehmen,
- einen piezoelektrischen Ultraschallwandler (219) mit einem piezoelektrischen Motor (220), der zwischen einer Gegenmasse (224) und einem proximalen Ende (226a) eines Verstärkerteils (226) angeordnet ist, wobei der piezoelektrische Wandler geeignet ist, Ultraschallwellen an den Einsatz von einem distalen Ende (226b) des in der Aufnahme (207) vorhandenen Verstärkerteils (226) aus zu übertragen, und
- Drehantriebsmittel (202, 204, 208), die in der Aufnahme vorhanden sind, um den Einsatz drehanzutreiben,
wobei die Drehantriebsmittel von dem piezoelektrischen Ultraschallwandler (219) unabhängig beweglich sind,
wobei das chirurgische Handstück **dadurch gekennzeichnet ist, dass** der piezoelektrische Ultraschallwandler gegenüber dem Handstück (200) statisch ist.

2. Handstück nach Anspruch 1, bei dem die Drehantriebsmittel einen Mikromotor (202) umfassen, der mittels einer Übertragungswelle (204) mit der Aufnahme verbunden ist.

3. Handstück nach Anspruch 2, wobei die Rotationsachse (A) der Drehantriebsmittel in der Aufnahme (207) zu einem distalen Teil (204b) der Übertragungswelle senkrecht verläuft.

4. Handstück nach Anspruch 2, bei dem die Rotationsachse (A) der Drehantriebsmittel in der Aufnahme (607) zu einem distalen Teil der Übertragungswelle (604) parallel verläuft.

5. Handstück nach Anspruch 4, bei dem der piezoelektrische Ultraschallwandler (619) dazu ausgelegt ist, die Ultraschallwellen mittels der Drehantriebsmittel an die Aufnahme (607) zu übertragen.

6. Handstück nach Anspruch 1, bei dem die Drehantriebsmittel eine Luftturbine (350) und eine Übertragungsleitung (354) umfassen, wobei die Turbine geeignet ist, unter der Wirkung eines über die Übertragungsleitung herausgespritzten Gasstroms in Rotation zu geraten.

7. Handstück nach Anspruch 6, wobei die Rotationsachse (A) der Drehantriebsmittel in der Aufnahme (307) zu einem distalen Teil (354b) der Übertragungsleitung (354) senkrecht verläuft.

8. Handstück nach Anspruch 6, wobei die Rotationsachse (A) der Drehantriebsmittel in der Aufnahme zu einem distalen Teil der Übertragungsleitung parallel verläuft

9. Handstück nach einem der Ansprüche 1 bis 8, bei dem der piezoelektrische Ultraschallwandler (219) ein Schwingungsübertragungselement (230) aus Keramik umfasst, das an dem distalen Ende (226b) des Verstärkerteils (226) angeordnet ist, um die Ultraschallwellen in der Aufnahme (207) zu übertragen.

10. Handstück nach einem der Ansprüche 1 bis 9, bei dem der Verstärkerteil (226) eine Einschnürung (226c) aufweist, deren Position entlang des Verstärkerteils derart festgelegt ist, dass der Ausschlag der Schwingungsbewegungen des distalen Endes (226b) des Verstärkerteils maximiert wird.

11. Handstück nach einem der Ansprüche 1 bis 10, ferner umfassend einen Einsatz (210), der teilweise in der Aufnahme (207) angeordnet ist, um mit dem piezoelektrischen Ultraschallwandler (219) und mit den Drehantriebsmitteln (202, 204, 208) zusammenwirken zu können.

12. Handstück nach Anspruch 11, bei dem der Einsatz (210) derart angeordnet ist, dass sein proximales Ende (210a) mit dem distalen Ende (226b) des Verstärkerteils (226) in Kontakt ist und dass der Körper des Einsatzes (210) durch die Drehantriebsmittel (202, 204, 208) drehangetrieben werden kann.

## Claims

1. A surgical handpiece (200) comprising:
• a housing (207) for receiving an insert (210);
• an ultrasound piezoelectric transducer (219) comprising a piezoelectric motor (220) placed between a counterweight (224) and a proximal end (226a) of an amplifier portion (226), the piezoelectric transducer being suitable for transmitting ultrasound waves from a distal end (226b) of said amplifier portion (226) to said insert, the distal end being present in the housing (207); and
• rotary drive means (202, 204, 208) present in said housing for driving in rotation said insert;
the rotary drive means being movable independently of the ultrasound piezoelectric transducer (219), the surgical handpiece being **characterized in that** said ultrasound piezoelectric transducer is static relative to the handpiece (200).

2. A handpiece according to claim 1, wherein the rotary drive means comprise a micromotor (202) connected to the housing via a transmission shaft (204).

3. A handpiece according to claim 2, wherein the axis of rotation (A) of the rotary drive means in said housing (207) is perpendicular to a distal portion (204b) of the transmission shaft.

4. A handpiece according to claim 2, wherein the axis of rotation (A) of the rotary drive means in said housing (607) is parallel to a distal portion of the transmission shaft (604).

5. A handpiece according to claim 4, wherein the ultrasound piezoelectric transducer (619) is configured to transmit ultrasound waves to said housing (607) via the rotary drive means.

6. A handpiece according to claim 1, wherein the rotary drive means comprise an air turbine (350) and a transmission duct (354), said turbine being suitable for entering into rotation under the action of a stream of gas blown via said transmission duct.

7. A handpiece according to claim 6, wherein the axis of rotation (A) of the rotary drive means in said housing (307) is perpendicular to a distal portion (354b) of the transmission duct (354).

8. A handpiece according to claim 6, wherein the axis of rotation (A) of the rotary drive means in said housing is parallel to a distal portion of the transmission duct.

9. A handpiece according to any one of claims 1 to 8, wherein the ultrasound piezoelectric transducer (219) includes a ceramic vibratory transmission element (230) arranged at the distal end (226b) of the amplifier portion (226) so as to transmit the ultrasound waves in said housing (207).

10. A handpiece according to any one of claims 1 to 9, wherein the amplifier portion (226) presents a constriction (226c) of position along the amplifier portion that is set in such a manner as to maximize the amplitude of the vibratory motion of the distal end (226b) of the amplifier portion.

11. A handpiece according to any one of claims 1 to 10, also including an insert (210) placed in part in the housing (207) so as to be capable of co-operating with the ultrasound piezoelectric transducer (219) and with the rotary drive means (202, 204, 208).

12. A handpiece according to claim 11, wherein the insert (210) is arranged in such a manner that its proximal end (210a) is in contact with the distal end (226b) of the amplifier portion (226), and that the body of the insert (210) is capable of being driven in rotation by the rotary drive means (202, 204, 208).
